# EUROPEAN PATENT APPLICATION

(11) **EP 4 817 670 A1**
(43) Date of publication of application: **07.10.2026**
(21) Application number: 24910608.9
(22) Date of filing: 05.12.2024
(51) Int. Cl.: A61M 60/861, A61M 60/863, A61M 60/865, A61M 60/165

(54) **VENTRICULAR CONNECTION MECHANISM AND VENTRICULAR ASSIST SYSTEM**

(30) Priority: 30.12.2023 CN 202311858924
(71) Applicant: Shenzhen Core Medical Technology Co., Ltd., Shenzhen, Guangdong 518000 (CN)
(72) Inventor: XIAO, Zhenzhong, Shenzhen, Guangdong 518000 (CN); YU, Shunzhou, Shenzhen, Guangdong 518000 (CN); XIE, Duanqing, Shenzhen, Guangdong 518000 (CN)
(74) Representative: Appleyard Lees IP LLP
(86) International application number: PCT/CN2024/137065
(87) International publication number: WO 2025/139676

(57) **Abstract**

A ventricular connection mechanism (1) and a ventricular assist system. The ventricular connection mechanism (1) comprises a suture ring (10) and a clamping assembly (20). The suture ring (10) is provided with an accommodating hole (13) allowing an inlet pipe of a ventricular assist device to pass through, and a first annular groove (111) surrounding the accommodating hole (13) is provided in the suture ring (10), to divide the suture ring (10) into a sealing portion (15) surrounding the accommodating hole (13) and a suture portion (14) surrounding the first annular groove (111); and the clamping assembly (20) comprises a clamping ring (21), wherein at least part of the clamping ring (21) is embedded in the first annular groove (111), the clamping ring (21) is sleeved on the sealing portion (15), the clamping ring (21) clamps the inlet pipe by means of the sealing portion (15), and the inner diameter of the clamping ring (21) is adjustable, so as to adjust the tightness of the sealing portion (15) clamping the inlet pipe.

## Description

### CROSS-REFERENCE TO RELATED APPLICATIONS

This application claims priority of Chinese Patent Application No. 202311858924.0, filed on December 30, 2023, the content of which is hereby incorporated by reference in its entirety.

### TECHNICAL FIELD

The present application relates to the technical field of medical devices, and in particular to a ventricular connection mechanism and a ventricular assist system.

### BACKGROUND

Ventricular assist devices can assist the heart in pumping blood and are commonly used to assist in the treatment of cardiovascular diseases. During the implantation surgery of ventricular assist devices, a ventricular connection mechanism is usually required to secure the ventricular assist device to heart tissue. However, conventional ventricular connection mechanisms are prone to blood leakage after the blood pump is mounted, which needs to be improved.

### SUMMARY

Based on this, the present application provides a ventricular connection mechanism and a ventricular assist system that can achieve a 360° seal on the inlet pipe along a circumferential direction thereof, so as to prevent a gap between a sealing portion and the inlet pipe and reduce the probability of blood leakage.

In a first aspect, a ventricular connection mechanism is provided according to an embodiment of the present application, and the ventricular connection mechanism includes:
a suture ring provided with an accommodating hole configured to allow an inlet pipe of a ventricular assist device to extend through, wherein the suture ring is provided with a first annular groove surrounding the accommodating hole, such that the suture ring is divided into a sealing portion surrounding the accommodating hole and a suturing portion surrounding the first annular groove; and
a clamping assembly including a clamping ring, wherein at least a portion of the clamping ring is embedded in the first annular groove, the clamping ring is sleeved on the sealing portion, the clamping ring clamps the inlet pipe through the sealing portion, and an inner diameter of the clamping ring is adjustable, so as to adjust a tightness of the sealing portion clamping the inlet pipe.

In a second aspect, a ventricular assist system is provided according to an embodiment of the present application. The ventricular assist system includes a ventricular assist device and the ventricular connection mechanism described above, and the ventricular assist device includes an inlet pipe extending through the accommodating hole.

Details of one or more embodiments of the present application are set forth in the following drawings and descriptions. Other features, objects and advantages of the present application will become apparent with reference to the specification, drawings, and claims.

### BRIEF DESCRIPTION OF THE DRAWINGS

In order to illustrate the embodiments of the present application more clearly, the drawings used in the embodiments will be described briefly. Apparently, the following described drawings are merely for the embodiments of the present application, and other drawings can be derived by those of ordinary skill in the art without any creative effort.
FIG. 1 is a schematic structural view of a ventricular connection mechanism provided in the present application from one perspective.
FIG. 2 is an exploded view of the ventricular connection mechanism shown in FIG. 1 from one perspective.
FIG. 3 is a schematic structural view of a suture ring of the ventricular connection mechanism shown in FIG. 1.
FIG. 4 is a top view of the ventricular connection mechanism shown in FIG. 1.
FIG. 5 is a cross-sectional view of the ventricular connection mechanism shown in FIG. 4 taken along the line I-I.
FIG. 6 is a partial enlarged view of part A in FIG. 5.
FIG. 7 is a back schematic view of a suture ring according to an embodiment.
FIG. 8 is another partial enlarged view of part A in FIG. 5.
FIG. 9 is a schematic structural view of a clamping ring and a locking assembly of the ventricular connection mechanism shown in FIG. 1.
FIG. 10 is a schematic structural view of a fixing ring of the ventricular connection mechanism shown in FIG. 1.
FIG. 11 is a cross-sectional view of the ventricular connection mechanism shown in FIG. 1 in a disassembled state.
FIG. 12 is a partial enlarged view of part B in FIG. 6.
FIG. 13 is a schematic structural view of another ventricular connection mechanism provided in the present application.
FIG. 14 is a partial enlarged view of part C in FIG. 13.
FIG. 15 is a schematic structural view of the ventricular connection mechanism shown in FIG. 13 from another perspective.

### DETAILED DESCRIPTION OF THE EMBODIMENTS

The present application will now be described in detail with reference to the accompanying drawings and embodiments in order to make the objects, technical solutions, and advantages of the present application clearer. It should be understood that the specific embodiments described herein are only for explaining the present application, and not intended to limit the present application.

It should be noted that when an element is called "fixed to" or "provided on" another element, it can be directly on another element or there can be a centered element. When an element is considered to be "connected" to another element, it can be directly connected to another element or indirectly connected to the other element.

In addition, the terms "first" and "second" are only used for descriptive purposes, and should not be construed as indicating or implying relative importance or implying the number of indicated technical features. Thus, a feature delimited with "first", "second" may expressly or implicitly include at least one of the features. In the description of the present application, "plurality" means two or more, unless otherwise expressly and specifically defined.

In order to illustrate the technical solution of the present application, the specific drawings and embodiments will be combined to illustrate below.

In blood pump implantation surgery, a ventricular connection mechanism is usually used to fix a blood pump to a heart. Specifically, the ventricular connection mechanism is fixed to an outer wall of the heart, an inlet pipe of the blood pump extends through the ventricular connection mechanism and extends into a ventricle, and the inlet pipe is locked by the ventricular connection mechanism. The inventors of the present application have discovered that, with conventional ventricular connection mechanisms, there is a risk of blood leaking from an opening of an annular disc. In addition, the conventional ventricular connection mechanisms have a relatively large thickness, resulting in a relatively high risk of the ventricular assist device mounted with the ventricular connection mechanism coming into contact with other organs within the thoracic cavity.

To address at least some of the above-mentioned problems, the present application provides a ventricular connection mechanism and a ventricular assist system. By configuring a suture ring to have a structure with a sealing portion surrounding an inner hole, and a clamping ring being sleeved on an outer periphery of the sealing portion, the sealing portion can be sealingly attached to the inlet pipe. Thus, by using the suture ring with a specific structure, the inlet pipe can be sealed 360° along a circumferential direction of the suture ring, thereby reducing the probability of blood leakage. In addition, the suture ring further includes a suturing portion surrounding the outer periphery of the sealing portion. By forming an annular groove surrounding the inner hole on the suture ring, the suture ring is divided into the sealing portion surrounding the accommodating hole and the suturing portion surrounding the annular groove, so as to avoid increasing an axial thickness of the suture ring due to the sealing portion being provided on a surface of the suture ring, so that the sealing portion and the suturing portion at least partially overlap in the axial direction of the suture ring. The thickness of the suture ring in the axial direction is reduced, a height of the ventricular assist device protruding from the outer surface of the heart after mounting is reduced, thereby reducing the risk of the ventricular assist device coming into contact with other organs within the thoracic cavity. Therefore, the ventricular connection mechanism of the present application can reduce the probability of blood leakage at the connection between the ventricular connection mechanism and the inlet pipe of the ventricular assist device while minimizing the increase in the thickness of the ventricular connection mechanism. The ventricular connection mechanism and ventricular assist system provided in the present application will be described in detail below with reference to specific embodiments and accompanying drawings.

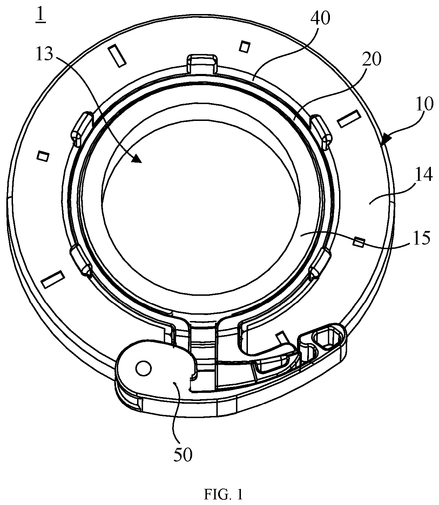

Referring to FIG. 1 to FIG. 4, an embodiment of the present application provides a ventricular connection mechanism 1, which is configured to fix a ventricular assist device to a heart tissue. The ventricular connection mechanism 1 includes a suture ring 10 and a clamping assembly 20.

The suture ring 10 is configured to be fixed to the heart tissue. Specifically, the suture ring 10 is provided with an accommodating hole 13 configured to allow an inlet pipe of the ventricular assist device to extend through. The suture ring 10 is provided with a first annular groove 111 surrounding the accommodating hole 13, such that the suture ring 10 is divided into a sealing portion 15 surrounding the accommodating hole 13 and a suturing portion 14 surrounding the first annular groove 111.

The clamping assembly 20 includes a clamping ring 21. At least a portion of the clamping ring 21 is embedded in the first annular groove 111, and the clamping ring 21 is sleeved on the outer periphery of the sealing portion 15. The clamping ring 21 clamps the inlet pipe through the sealing portion 15. An inner diameter of the clamping ring 21 is adjustable, that is, the clamping ring 21 can contract radially inward under the action of external force to adjust its inner diameter, so as to adjust the tightness of the sealing portion 15 clamping the inlet pipe. The radial direction of the clamping ring 21 refers to a direction along a radius or diameter of the clamping ring 21. In this embodiment, the clamping ring 21 is entirely embedded within the first annular groove 111. In other embodiments, the clamping ring 21 may be partially located within the first annular groove 111 and partially located outside the first annular groove 111 and protrude from the surface of the suture ring 10.

In the aforementioned ventricular connection mechanism 1, the suture ring 10 is provided with the accommodating hole 13 through which the inlet pipe of the ventricular assist device extends, and the suture ring 10 is provided with the first annular groove 111 surrounding the accommodating hole 13, so as to form the sealing portion 15 surrounding the accommodating hole 13. Furthermore, at least a portion of the clamping ring 21 is embedded in the first annular groove 111, so that the clamping ring 21 is sleeved on the outer periphery of the sealing portion 15. Thus, by adjusting the inner diameter of the clamping ring 21, the diameter of the accommodating hole 13 can be reduced to adjust the tightness of the sealing portion 15 clamping the inlet pipe, so that the sealing portion 15 is sealingly attached to the inlet pipe, so as to achieve a 360° sealing on the inlet pipe along the circumferential direction of the suture ring 10, prevent a gap between the sealing portion 15 and the inlet pipe, and reduce the probability of blood leakage. The circumferential direction of the suture ring 10 refers to a circular direction of the suture ring 10.

At least a portion of the clamping ring 21 is embedded in the first annular groove 111 formed in the suture ring 10, so that the clamping ring 21 and the suture ring 10 are at least partially overlapped along the axial direction of the suture ring 10, which reduces the thickness of the ventricular connection mechanism 1 at the suture ring 10, thereby reducing the height of the ventricular assist device protruding from the outer surface of the heart after mounting, and thus reducing the risk of the ventricular assist device coming into contact with other organs in the thoracic cavity. In this embodiment, the axial direction of the suture ring 10 refers to a direction consistent with an extension direction of a central axis of the suture ring 10.

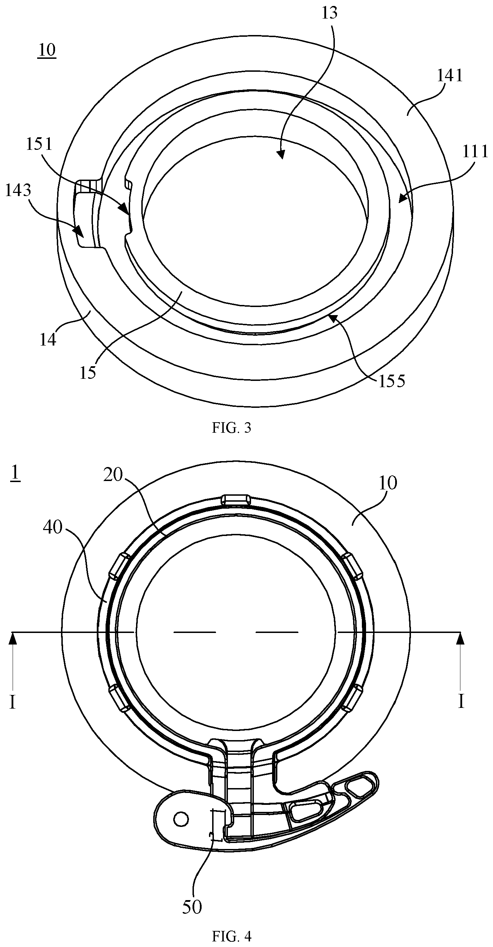

Referring to FIG. 1 and FIG. 3, the suture ring 10 may be made of a soft rubber with good biocompatibility, such as silicone, etc., so that the suture ring 10 can be easily attached to the heart tissue and the suture thread can easily extend through the suture ring 10. Furthermore, the ventricular connection mechanism 1 may further include a suturing cloth (not shown) covering the surfaces of the suture ring 10 and the clamping ring 21, thus enabling the suture ring 10 and the clamping ring 21 to be more tightly joined. Furthermore, the suture thread extends through the suturing cloth and the suturing portion 14 of the suture ring 10 and is sutured to the heart tissue, thereby fixing the ventricular connection mechanism 1 to the heart tissue. Preferably, the suturing cloth is made of a textile fabric, such as polyester fabric, etc.

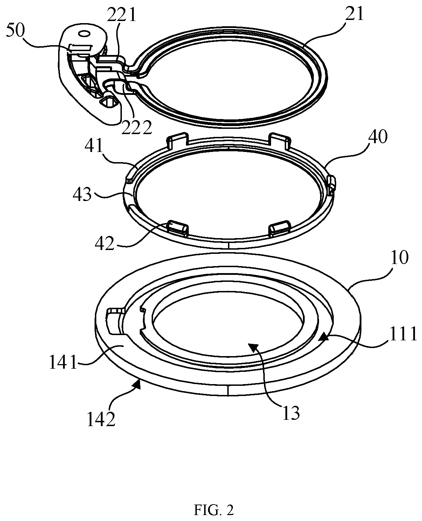

Referring to FIG. 1 and FIG. 2, the suture ring 10 has a suturing top surface 141 and a suturing bottom surface 142 opposite to the suturing top surface 141. The suturing bottom surface 142 can conform to the outer wall of the heart. The first annular groove 111 is exposed on the suturing top surface 141, that is, the first annular groove 111 is recessed from the suturing top surface 141 toward the suturing bottom surface 142. The first annular groove 111 is of an annular groove structure. The first annular groove 111 is provided surrouding the outer periphery of the sealing portion 15, and at least a portion of the clamping ring 21 is embedded in the first annular groove 111. Thus, the clamping ring 21 and the suture ring 10 are more tightly combined. Compared with conventional clamping ring and suture ring connected by suturing, by embedding at least a portion of the clamping ring 21 in the first annular groove 111, the problem of blood leakage caused by an increase in the gap between the clamping ring 21 and the suture ring 10 can be avoided as much as possible during the pulling process when the suture ring is sutured to the heart.

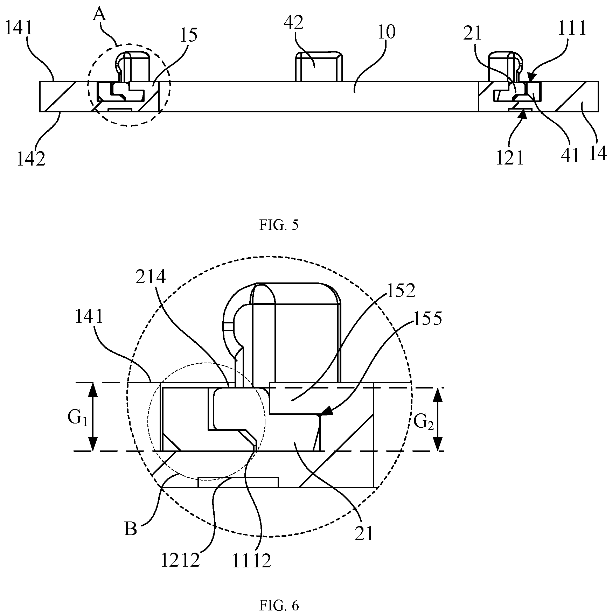

Referring to FIG. 5 and FIG. 6, the first annular groove 111 has a groove bottom surface 1112. A first axial distance G1 is defined between the groove bottom surface 1112 and the suturing top surface 141. The clamping ring 21 has a ring top surface 214 opposite to the groove bottom surface 1112. A second axial distance G2 is defined between the ring top surface 214 and the groove bottom surface 1112. In this embodiment, the second axial distance G2 is less than or equal to the first axial distance G1, that is, the clamping ring 21 will not protrude outward after being mounted in the first annular groove 111, so that the thickness of the suture ring 10 is the thickness of the ventricular connection mechanism 1 at the suture ring 10, which further reduces the overall axial height of the suture ring 10 and further reduces the risk of the ventricular assist device coming into contact with other organs within the thoracic cavity. The first axial distance G1 and the second axial distance G2 both refer to distances along the axial direction of the suture ring 10.

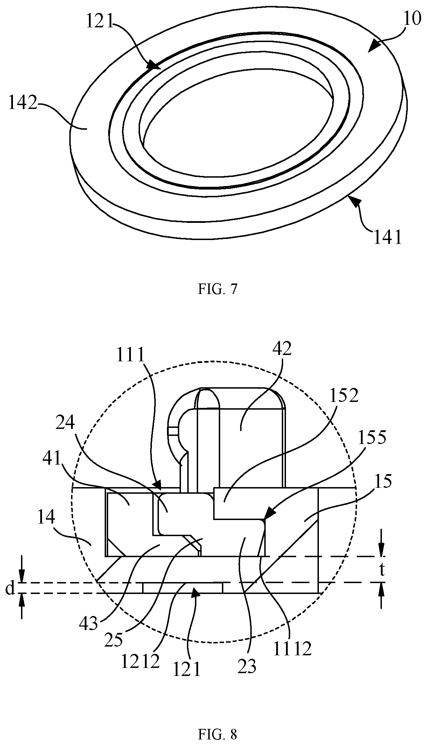

Referring to FIG. 7 and FIG. 8, in an embodiment, the suture ring 10 is further provided with a second annular groove 121. The first annular groove 111 and the second annular groove 121 are provided on opposite sides of the suture ring 10, that is, the second annular groove 121 is exposed on the suturing bottom surface 142. The second annular groove 121 is an annular groove structure, and the first annular groove 111 and the second annular groove 121 are corresponding and spaced apart along the axial direction of the suture ring 10. The first annular groove 111 and the second annular groove 121 are spaced apart along the axial direction of the suture ring 10, which means that a soft rubber with a certain thickness is provided between a bottom surface of the first annular groove 111 and a bottom surface of the second annular groove 121, that is, the two bottom surfaces are not connected. The second annular groove 121 and the first annular groove 111 are corresponding along the axial direction of the suture ring 10, which means that an orthographic projection of an edge of the second annular groove 121 on the suturing top surface 141 is located within the first annular groove 111, or means that an orthographic projection of an edge of the first annular groove 111 on the suturing bottom surface 142 is located within the second annular groove 121. The orthogonal projection refers to a projection along the axial direction of the suture ring 10.

By forming the second annular groove 121 on the suturing bottom surface 142 corresponding to the position of the first annular groove 111, the thickness of the suture ring 10 at the position corresponding to the first annular groove 111 can be reduced, which is beneficial for improving the elastic deformation capability of the sealing portion 15, causing the sealing portion 15 to more easily contract inward under the clamping action of the clamping ring 21, thereby facilitating the sealing portion 15 to be sealingly attached to the inlet pipe and avoiding the situation where the sealing portion 15 cannot contract inward.

In this embodiment, a depth of the second annular groove 121 is defined as d, and the minimum distance between a bottom surface 1112 of the first annular groove 111 and a bottom surface 1212 of the second annular groove 121 is defined as t. That is, the suture ring 10 has a minimum thickness t at the first annular groove 111, where: 2≤d/t≤0.4. In this way, while ensuring the connection strength between the sealing portion 15 and the suturing portion 14, the sealing portion 15 can more easily contract inward under the clamping action of the clamping ring 21, so that it is convenient for medical staff to operate the clamping ring 21 to grip the inlet pipe of the ventricular assist device. When d/t < 0.2, the depth of the second annular groove 121 is too small, or the thickness of the suture ring 10 at the position corresponding to the first annular groove 111 is too large, which is unfavorable for the inward contraction of the sealing portion 15. When d/t > 0.4, the depth of the second annular groove 121 is too large, or the thickness of the suture ring 10 at the position corresponding to the first annular groove 111 is too small, which can easily lead to breakage at the connection between the sealing portion 15 and the suturing portion 14.

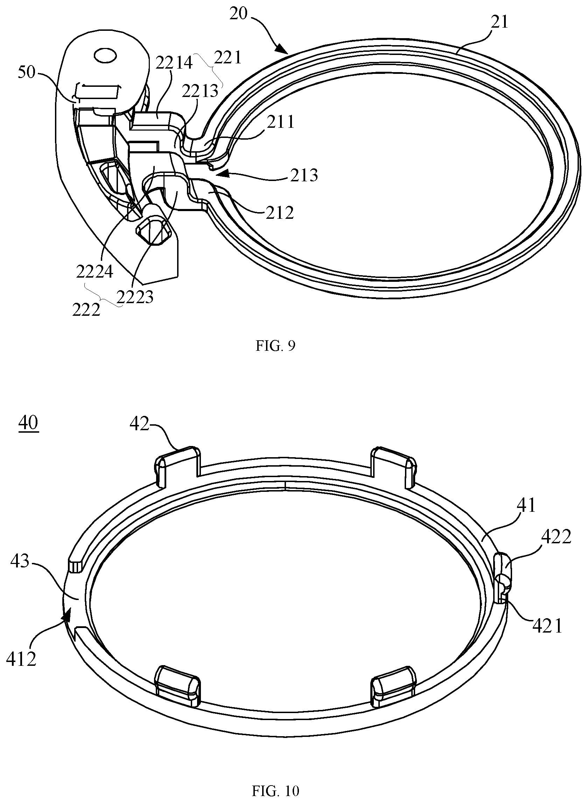

Referring to FIG. 9, in an embodiment, the clamping assembly 20 further includes two connecting arms connected to the clamping ring 21. The two connecting arms can move toward each other or away from each other, so as to adjust the inner diameter of the clamping ring 21. Each connecting arm has a support pillar and an extension portion. The support pillar extends along an axial direction of the clamping ring 21. One end of the support pillar is connected to the clamping ring 21. The extension portion extends from an end of the support pillar away from the clamping ring 21 in a direction away from the clamping ring 21 along the radial direction of the clamping ring 21. The extension portion is configured to be connected to a locking assembly, or for an operator to grip by hand or with tools to adjust a distance between the two connecting arms, thereby adjusting the inner diameter of the clamping ring 21.

Specifically, in the illustrated embodiment, the clamping ring 21 has an opening 213 formed along the circumferential direction of the clamping ring 21, and a first end 211 and a second end 212 that form the opening 213. The first end 211 and the second end 212 are provided opposite to each other along the circumferential direction of the clamping ring 21. The first end 211 and the second end 212 are spaced apart to form the opening 213. By adjusting the size of the opening 213, the inner diameter of the clamping ring 21 can be adjusted. The two connecting arms are fixedly connected to the first end 211 and the second end 212, respectively. The circumferential direction of the clamping ring 21 refers to the circumferential direction of the clamping ring 21, and in this embodiment, the circumferential direction of the clamping ring 21 is consistent with the circumferential direction of the suture ring 10.

In this embodiment, the two connecting arms are a first connecting arm 221 and a second connecting arm 222. The first connecting arm 221 is fixed to the first end 211, and the second connecting arm 222 is fixed to the second end 212.

The first connecting arm 221 includes a first support pillar 2213 and a first extension portion 2214. The first support pillar 2213 is connected between the first end 211 and the first extension portion 2214. The first support pillar 2213 extends along the radial direction of the clamping ring 21. The first extension portion 2214 extends from an end of the first support pillar 2213 away from the first end 211 in a direction away from the clamping ring 21 along the radial direction of the clamping ring 21

The second connecting arm 222 includes a second support pillar 2223 and a second extension portion 2224. The second support pillar 2223 is connected between the second end 212 and the second extension portion 2224. The second support pillar 2223 extends the radial direction of the clamping ring 21. The second extension portion 2224 extends from an end of the second support pillar 2223 away from the second end 212 in a direction away from the clamping ring 21 along the radial direction of the clamping ring 21.

Referring to FIGS. 3 and 9, in one embodiment, a first avoidance groove 143 is provided on the suturing portion 14. The first avoidance groove 143 is in communication with the first annular groove 111, and the first avoidance groove 143 is exposed on the suturing top surface 141. The ends of the support pillars of the two connecting arms that are connected to the clamping ring 21 are accommodated in the first avoidance groove 143, while the extension portions are located outside the first avoidance groove 143, that is, the end of the first support pillar 2213 and the end of the second support pillar 2223 that are connected to the clamping ring 21 are accommodated in the first avoidance groove 143, and both the first extension portion 2214 and the second extension portion 2224 are located outside the first avoidance groove 143, so as to facilitate connection with the locking assembly or for the operator to grip with hands or tools. In this embodiment, the first support pillar 2213 and the second support pillar 2223 both extend in a direction away from the suturing bottom surface 142 along the axial direction of the suture ring 10, and the first extension portion 2214 and the second extension 2224 are spaced apart from the suturing portion 14 in the axial direction of the suture ring 10, thereby avoiding the first connecting arm 221 and the second connecting arm 222 from colliding with the suturing portion 14 when the clamping ring 21 contracts (i.e., the first connecting arm 221 and the second connecting arm 222 approach each other), thus ensuring that the clamping ring 21 can smoothly contract radially inward.

Referring to FIGS. 3, 6, and 8, the sealing portion 15 is substantially of an annular structure. The inner peripheral surface of the sealing portion 15 is the wall of the accommodating hole 13, and the outer peripheral surface of the sealing portion 15 is the groove wall of a side of the first annular groove 111 adjacent to the accommodating hole 13.

Furthermore, the outer peripheral surface of the sealing portion 15 is provided with a first flange 152 extending radially outward from the sealing portion 15. The first flange 152 is of an annular structure. The first flange 152 is spaced apart from the groove bottom surface 1112 of the first annular groove 111 to form a first slot 155. At least a portion of the clamping ring 21 is provided in the first slot 155 between the first flange 152 and the groove bottom surface 1112 of the first annular groove 111. Specifically, the inner periphery of the clamping ring 21 is provided with a first step 23, which is of an annular structure and protrudes inward along the radial direction of the clamping ring 21. The first step 23 is inserted into the first slot 155, that is, the first step 23 abuts between the first flange 152 and the groove bottom surface 1112. In other words, at least a portion of the first step 23 is accommodated in the first slot 155, and the first step 23 is clamped between the first flange 152 and the groove bottom surface 1112. Thus, the first flange 152 can restrict the clamping ring 21 along the axial direction of the suture ring 10, thereby enhancing the connection strength between the suture ring 10 and the clamping ring 21, and preventing the clamping ring 21 from detaching from the suture ring 10 during operation.

Furthermore, the sealing portion 15 is provided with a notch 151, the position of the notch 151 corresponds to the position of the opening 213 of the clamping ring 21. The position of the notch 151 corresponds to the position of the opening 213 of the clamping ring 21, which means that a width of the notch 151 is greater than a width of the opening 213, and an extension line of the edge of the opening 213 along the radial direction of the clamping ring 21 is within the edge of the notch 151. Alternatively, a width of the opening 213 is greater than a width of the notch 151, and an extension line of the edge of the notch 151 along the radial direction of the clamping ring 21 is within the edge of the opening 213. Alternatively, a width of the opening 213 is equal to a width of the notch 151.

The position of the notch 151 corresponds to the position of the opening 213 of the clamping ring 21, and the notch 151 is provided on the side of the sealing portion 15 away from the accommodating hole 13. Thus, when the inner diameter of the clamping ring 21 decreases, the sealing portion 15 at the notch 151 can be deformed toward the accommodating hole 13, which avoids the problem of a gap between the suture ring 10 and the inlet pipe of the ventricular assist device caused by the sealing portion 15 being deformed outwardly at the position corresponding to the notch 151, thereby ensuring the sealing performance of the suture ring 10 on the inlet pipe and further reducing the risk of blood leakage.

In this embodiment, the notch 151 is provided on the first flange 152 and extends along the circumferential direction of the suture ring 10. The notch 151 extends through both opposite sides of the first flange 152 along the axial direction of the first flange 152. The axial direction of the first flange 152 is consistent with the axial direction of the suture ring 10. In other embodiments, when the sealing portion 15 is not provided with the first flange 152, the notch 151 may alternatively be provided on an annular wall of the sealing portion 15, as long as it is provided on the side of the sealing portion 15 away from the accommodating hole 13.

Referring to FIGS. 4, 10, and 11, in an embodiment, the ventricular connection mechanism 1 further includes a fixing ring 40 provided on the suture ring 10. The suture ring 40 can be gripped by a clamp (not shown), so that the clamp can clamp the ventricular connection mechanism 1.

The retaining ring 40 includes a ring body 41 and a protruding buckle 42. The ring body 41 is an annular structure. The ring body 41 is fixed to the suture ring 10 and surrounds the clamping ring 21. The protruding buckle 42 is provided on the ring body 41 and protrudes from the ring body 41 to be gripped by the clamp, so that the clamp can firmly grip the ventricular connection mechanism 1, thereby facilitating the implantation of the ventricular assist device.

The protruding buckle 42 includes a main body 421 and a protruding portion 422. The main body 421 is connected to a top end of the ring body 41 and extends along the axial direction of the ring body 41. The top end of the ring body 41 refers to the end of the ring body 41 away from the groove bottom surface 1112 of the first annular groove 111. The axial direction of the ring body 41 is consistent with the axial direction of the suture ring 10. The protruding portion 422 is connected to the end of the main body 421 away from the ring body 41 and protrudes outward from the main body 421 along the radial direction of the clamping ring 21. The protruding portion 422 is configured to be engaged with the clamp, so as to improve the stability of the clamp in gripping the ventricular connection mechanism 1. In the illustrated embodiment, the main body 421 is of a rectangular columnar structure, and the protruding portion 422 is an arc-shaped protrusion. In other embodiments, the main body 421 may be of a cylindrical structure, and the protruding portion 422 may be a triangular protrusion, which is not limited herein.

In this embodiment, a plurality of protruding buckles 42 are provided. The plurality of protruding buckles 42 are spaced apart along the circumferential direction of the ring body 41. The circumferential direction of the ring body 41 is a circular direction of the ring body 41. Furthermore, the plurality of protruding buckles 42 are evenly distributed along the circumferential direction of the ring body 41, i.e., central angles between two adjacent protruding buckles 42 are equal. For example, in one embodiment, five protruding buckles 42 are provided on the ring body 41, and the central angle between two adjacent protruding buckles 42 is 72°, so as to ensure that the fixed ring 40 is subjected to more uniform force when the clamp grips the fixing ring 40. In another embodiment, the multiple protruding buckles 42 may be unevenly distributed along the circumferential direction of the ring body 41, i.e., the central angles between two adjacent protruding buckles 42 are not equal, which is not limited herein.

In other embodiments, one protruding buckle 42 may be provided. The protruding buckle 42 may be of an annular structure and may be gripped by the clamp, thereby facilitating the implantation of the ventricular assist device. In other embodiments, two protruding buckles 42 may be provided, and the two protruding buckles 42 may be substantially semi-annular.

In one embodiment, the protruding buckle 42 and the ring body 41 are integrally formed, for example, the buckle 42 and the ring 41 are integrally injection molded, which increases the connection strength between the protruding buckle 42 and the ring body 41. In another embodiment, the protruding buckle 42 may also be fixedly connected to the top end of the ring body 41 by welding or adhesive bonding.

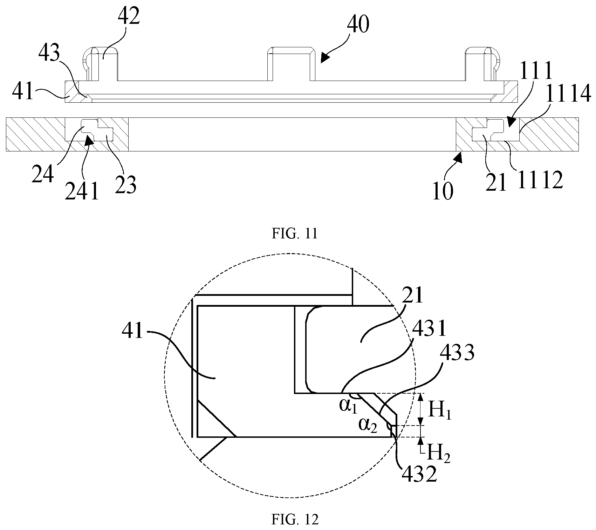

Referring to FIGS. 5 and 11, in this embodiment, both the ring body 41 of the fixing ring 40 and the clamping ring 21 are mounted in the first annular groove 111. In this way, it can avoid additionally forming a groove in the suturing area outside the first annular groove 111 to mount the ring body 41, thus ensuring the width of the suturing area of the suturing portion 14, which is beneficial for a doctor to suture the suturing portion 14 to the heart. In addition, the ring body 41 of the fixing ring 40 and the clamping ring 21 are both mounted in the first annular groove 111, which avoids the need for additional grooves and simplifies the manufacturing process of the ventricular connection mechanism 1.

In other embodiments, the ring body 41 of the fixing ring 40 and the clamping ring 21 may also be mounted at different positions, so that the mounting of the ring body 41 and the clamping ring 21 do not affect each other, which facilitates the mounting of the ring body 41 and the clamping ring 21. For example, the clamping ring 21 is mounted in the first annular groove 111, and the ring body 41 is fixed to the suturing top surface 141. Alternatively, the ring body 41 is fixed in another groove different from the first annular groove 111, as long as it can be gripped by the clamp.

In this embodiment, the ring body 41 is provided in the first annular groove 111 and sleeved on the clamping ring 21. The protruding buckle 42 extends along the axial direction of the ring body 41 and protrudes out of the first annular groove 111.

Furthermore, the first annular groove 111 further has a groove side surface 1114, which is located on a side of the groove bottom surface 1112 and faces the sealing portion 15. The groove side surface 1114 is spaced apart from and opposite to the sealing portion 15. The groove side surface 1114 is the inner peripheral surface of the suturing portion 14. The outer periphery of the clamping ring 21 is provided with a second flange 24, which extends from the outer periphery of the clamping ring 21 toward the groove side surface 1114. The second flange 24 is of an annular structure and surrounds the first step 23. The extension direction of the second flange 24 is opposite to the extension direction of the first step 23, i.e., the second flange 24 extends toward the ring body 41 of the fixing ring 40.

The second flange 24 is spaced apart from the groove bottom surface 1112 of the first annular groove 111 to form a second slot 241. The ring body 41 of the fixing ring 40 is provided between the second flange 24 and the groove side surface 1114. The inner peripheral surface of the fixing ring 40 is further provided with a second step 43. In this embodiment, the second step 43 is of an annular structure.

The second step 43 is inserted into the second slot 241, i.e., the second step 43 abuts between the second flange 24 and the groove bottom surface 1112 of the first annular groove 111, or in other words, the second step 43 is at least partially accommodated in the second slot 241, and the second step 43 is clamped between the second flange 43 and the groove bottom surface 1112 of the first annular groove 111. Thus, the second flange 24 can restrict the fixing ring 40 along the axial direction of the suture ring 10, so that the clamping ring 21 can press the fixing ring 40 tightly into the first annular groove 111, which enhances the connection strength between the fixing ring 40 and the suture ring 10 and prevents the fixing ring 40 from detaching from the suture ring 10 during operation. In one embodiment, the protruding buckle 42, the ring body 41, and the second step 43 are integrally formed.

Referring to FIGS. 7, 8, and 12, the clamping ring 21 further has a connecting portion 25 connecting the first step 23 and the second flange 24. The connecting portion 25, the first step 23, and the second flange 24 cooperatively form the clamping ring 21.

The second step 43 includes a step surface 431, a step side surface 432, and a beveled surface 433. The step surface 431 is opposite to the groove bottom surface 1112, the step side surface 432 is opposite to the groove side surface 1114, and the beveled surface 433 is connected between the step surface 431 and the step side surface 432.

In this embodiment, an angle α₁ formed between the beveled surface 433 and the step surface 431 is greater than 90°, and an angle α₂ formed between the beveled surface 433 and the step side surface 432 is greater than 90°. A height H₁ of the beveled surface 433 along the axial direction of the fixing ring 40 is greater than a height H₂ of the step side surface 431 along the axial direction of the fixing ring 40, so as to avoid the connecting portion 25. The axial direction of the fixing ring 40 is consistent with the axial direction of the suture ring 10. Thus, the first step 23 of the clamping ring 21 can be embedded into the first slot 155 (see FIG. 3), which prevents the clamping ring 21 from colliding with the fixing ring 40 during mounting, and facilitates the mounting of the clamping ring 21 after the fixing ring 40 is mounted.

Continuing to refer to FIGS. 3, 9, and 10, further, the ring body 41 is provided with a second avoidance groove 412, which extends through the inner and outer sides of the ring body 41 along the radial direction of the clamping ring 21, and the second avoidance groove 412 is correspondingly in communication with the first avoidance groove 143. The first connecting arm 221 and the second connecting arm 222 are provided in the second avoidance groove 412. In this way, collision between the first connecting arm 221 and the second connecting arm 222 with the fixing ring 40 is avoided when they approach each other, thereby ensuring that the clamping ring 21 can smoothly contract radially inward.

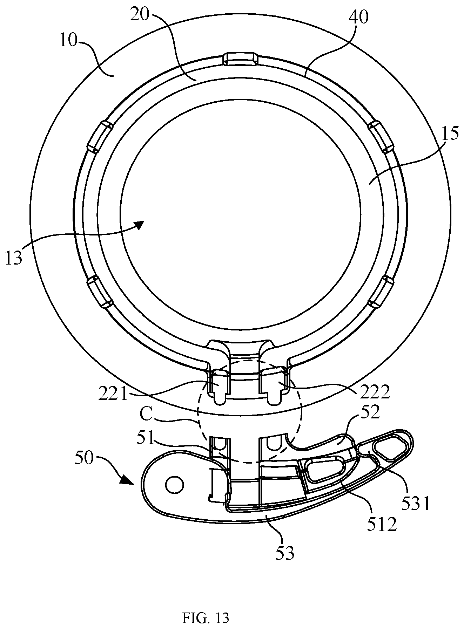

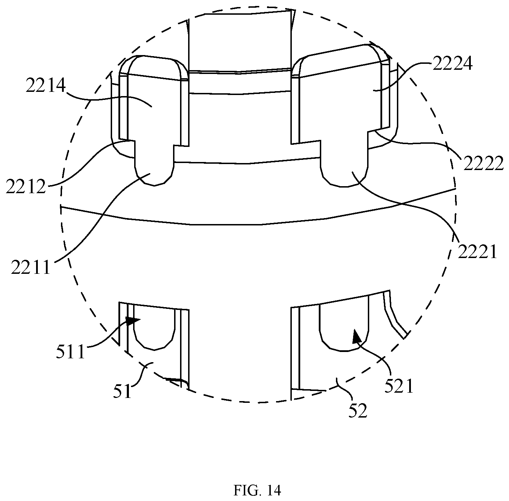

Referring to FIGS. 13 to 15, optionally, in an embodiment, the ventricular connection mechanism 1 further includes a locking assembly 50, which can drive the first connecting arm 221 and the second connecting arm 222 to move toward or away from each other. When the locking assembly 50 drives the first connecting arm 221 to approach the second connecting arm 222, the clamping ring 21 contracts, and the contraction of the clamping ring 21 can reduce the diameter of the accommodating hole 13, so that the sealing portion 15 is sealingly attached to the inlet pipe, i.e., the inner wall of the sealing portion 15 is tightly attached to the outer wall of the inlet pipe.

The locking assembly 50 includes a fixing base 51, a moving base 52, and a locking member 53. The fixing base 51 is connected to the first connecting arm 221, the moving base 52 is connected to the second connecting arm 222, and the moving base 52 is movably engaged with the fixing base 51. One end of the locking member 53 is connected to the fixing base 51, and the other end of the locking member 53 can be connected to or separated from the moving base 52. The fixing base 51 and the moving base 52 are connected to the extension portions of the two connecting arms, respectively. Specifically, the fixing base 51 and the moving base 52 are connected to the first extension portion 2214 of the first connecting arm 221 and the second extension portion 2224 of the second connecting arm 222, respectively.

Specifically, one end of the locking member 53 is rotatably connected to the fixing base 51, and the other end of the locking member 53 is provided with an engaging portion 531. The moving base 52 is provided with a curved surface 512. The engaging portion 531 is configured to cooperate with the curved surface 512 of the moving base 52. When the locking member 53 is driven to rotate relative to the fixing base 51, the engaging portion 531 is in contact with the curved surface 512 of the moving base 52 and pushes the moving base 52 to move toward the fixing base 51, thereby driving the second connecting arm 222 to move toward the first connecting arm 221, so that the clamping ring 21 contracts radially inward. When the clamping ring 21 contracts to clamp the inlet pipe, the engaging portion 531 is engaged with the curved surface 512 of the moving base 52 to maintain the clamping ring 21 in a contracted state. It should be understood that there are many other ways for the locking assembly 50 to drive the first connecting arm 221 and the second connecting arm 222 to move toward each other, which are not limited to the above embodiments and will not be described in detail.

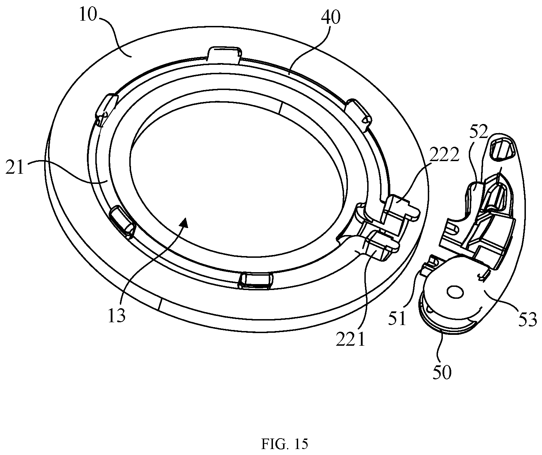

In one embodiment, the first connecting arm 221 and the fixing base 51 can be fixed by welding or adhesive bonding. As shown in FIG. 15, one of the first connecting arm 221 and the fixing base 51 is provided with a first protrusion 2211, and the other thereof is provided with a first groove 511. The first protrusion 2211 is embedded in the first groove 511, so that the first connecting arm 221 and the fixing base 51 are connected. On this basis, the first connecting arm 221 and the fixing base 51 can also be fixed by welding or adhesive bonding. In this embodiment, the end of the first connecting arm 221 is provided with the first protrusion 2211, and the fixing base 51 is provided with the first groove 511. The first protrusion 2211 is embedded in the first groove 511, so that the first groove 511 has a certain limiting effect on the first protrusion 2211, thereby improving the connection strength between the first connecting arm 221 and the fixing base 51. It should be understood that in other embodiments, the first protrusion 2211 may also be provided on the fixing base 51, and correspondingly, the first groove 511 may be provided on the first connecting arm 221, which can also increase the connection strength between the first connecting arm 221 and the fixing base 51.

Furthermore, the first connecting arm 221 has a first inclined surface 2212 opposite to the first end 211, and the first inclined surface 2212 is inclined relative to the extension direction of the first connecting arm 221. The first inclined surface 2212 is engaged with a surface of the fixing base 51 facing the clamping ring 21. Specifically, the first inclined surface 2212 is in contact with and engaged with the fixing base 51, and then the first connecting arm 221 is fixedly connected to the fixing base 51 by welding or adhesive bonding. The first inclined surface 2212 is in contact with and engaged with the fixing base 51, so that the contact area between the first connecting arm 221 and the fixing base 51 can be increased, which is beneficial to further strengthen the connection between the first connecting arm 221 and the fixing base 51.

In an embodiment, the second connecting arm 222 and the moving base 52 can be fixed by welding or adhesive bonding. As shown in FIG. 15, one of the second connecting arm 222 and the moving base 52 is provided with a second protrusion 2221, and the other is provided with a second groove 521. The second protrusion 2221 is embedded in the second groove 521, so that the second connecting arm 222 and the moving base 52 are connected. On this basis, the second connecting arm 222 and the moving base 52 can also be fixed by welding or adhesive bonding. In this embodiment, the end of the second connecting arm 222 is provided with the second protrusion 2221, and the moving base 52 of the locking assembly 50 is provided with the second groove 521. The second protrusion 2221 is embedded in the second groove 521, so that the second groove 521 has a certain limiting effect on the second protrusion 2221, thereby improving the connection strength between the second connecting arm 222 and the moving base 52. It should be understood that in other embodiments, the second protrusion 2221 may also be provided on the moving base 52, and the second groove 521 may be correspondingly provided on the second connecting arm 222, which can also strengthen the connection between the second connecting arm 222 and the moving base 52.

Furthermore, the second connecting arm 222 has a second inclined surface 2222 opposite to the second end 212, and the second inclined surface 2222 is inclined relative to the extension direction of the second connecting arm 222. The second inclined surface 2222 is engaged with a surface of the moving base 52 facing the clamping ring 21. Specifically, the second inclined surface 2222 is in contact with and engaged with the moving base 52, and then the second connecting arm 222 is fixedly connected to the moving base 52 by welding or adhesive bonding. The second inclined surface 2222 is in contact with and engaged with the moving base 52, so that the contact area between the second connecting arm 222 and the moving base 52 can be increased, which is beneficial to strengthen the connection between the second connecting arm 222 and the moving base 52.

The present application also provides a ventricular assist system, which includes a ventricular assist device (not shown) and a ventricular connection mechanism 1 according to any one of the above embodiments. The ventricular assist device includes an inlet pipe extending through the accommodating hole 13. Thus, by driving the clamping ring 21 to contract radially inward through the locking assembly 50, the suture ring 10 can be driven to contract, thereby causing the suture ring 10 to clamp the inlet pipe along the circumferential direction of the suture ring 10, thus sealing the inlet pipe and reducing the risk of blood leakage.

The above embodiments are only used to illustrate the technical solutions of the present application and are not intended to limit the present application. Although the present application has been described in detail with reference to the foregoing embodiments, those skilled in the art should understand that modifications can still be made to the technical solutions described in the foregoing embodiments, or equivalent substitutions can be made to some of the technical features. Such modifications or substitutions do not cause the essence of the corresponding technical solutions to deviate from the spirit and scope of the technical solutions of the embodiments of the present application, and should all fall within the protection scope of the present application.

## Claims

1. A ventricular connection mechanism, comprising:
a suture ring provided with an accommodating hole configured to allow an inlet pipe of a ventricular assist device to extend through, wherein the suture ring is provided with a first annular groove surrounding the accommodating hole, such that the suture ring is divided into a sealing portion surrounding the accommodating hole and a suturing portion surrounding the first annular groove; and
a clamping assembly comprising a clamping ring, wherein at least a portion of the clamping ring is embedded in the first annular groove, the clamping ring is sleeved on the sealing portion , the clamping ring clamps the inlet pipe through the sealing portion, and an inner diameter of the clamping ring is adjustable, so as to adjust a tightness of the sealing portion clamping the inlet pipe.

2. The ventricular connection mechanism according to claim 1, wherein the suture ring has a suturing bottom surface and a suturing top surface opposite to the suturing bottom surface, the suturing bottom surface is capable of conforming to an outer wall of a heart, the first annular groove is exposed on the suturing top surface, the first annular groove has a groove bottom surface, a first axial distance is defined between the groove bottom surface and the suturing top surface, the clamping ring has a ring top surface opposite to the groove bottom surface, a second axial distance is defined between the ring top surface and the groove bottom surface, and the second axial distance is less than or equal to the first axial distance.

3. The ventricular connection mechanism according to claim 1, wherein the suture ring is further provided with a second annular groove, the first annular groove and the second annular groove are provided on opposite sides of the suture ring, and the first annular groove and the second annular groove are corresponding and spaced apart along an axial direction of the suture ring.

4. The ventricular connection mechanism according to claim 3, wherein a depth of the second annular groove is defined as d, a minimum distance between a groove bottom surface of the first annular groove and a groove bottom surface of the second annular groove is defined as t, and 0.2≤d/t≤0.4.

5. The ventricular connection mechanism according to claim 1, wherein an outer peripheral surface of the sealing portion is provided with a first flange extending radially outward from the sealing portion, the first flange and a groove bottom surface of the first annular groove are spaced apart to form a first slot, an inner periphery of the clamping ring is provided with a first step, and the first step is inserted into the first slot.

6. The ventricular connection mechanism according to claim 1, further comprising a fixing ring, wherein the fixing ring comprises a ring body and a protruding buckle, the ring body is fixed to the suture ring and surrounds the clamping ring, and the protruding buckle protrudes from the ring body and is configured to be gripped by a clamp.

7. The ventricular connection mechanism according to claim 6, wherein the suture ring has a suturing bottom surface and a suturing top surface opposite to the suturing bottom surface, the suturing bottom surface is capable of conforming to an outer wall of a heart, the first annular groove is exposed on the suturing top surface, both the ring body of the fixing ring and the clamping ring are mounted in the first annular groove.

8. The ventricular connection mechanism according to claim 7, wherein an outer peripheral surface of the sealing portion is provided with a first flange extending radially outward from the sealing portion, the first flange and a groove bottom surface of the first annular groove are spaced apart to form a first slot, an inner periphery of the clamping ring is provided with a first step, the first step is inserted into the first slot, the first annular groove further has a groove side surface spaced apart from and opposite to the sealing portion, an outer periphery of the clamping ring is provided with a second flange extending toward the groove side surface, the second flange is spaced apart from the groove bottom surface of the first annular groove to form a second slot, the ring body of the fixing ring is provided between the second flange and the groove side surface, an inner peripheral surface of the ring body is further provided with a second step, and the second step is inserted into the second slot.

9. The ventricular connection mechanism according to claim 8, wherein the clamping ring has a connecting portion connecting the first step and the second flange, the second step comprises a step surface opposite to the groove bottom surface, a step side surface opposite to the groove side surface, and a beveled surface connecting the step surface and the step side surface, an angle formed between the beveled surface and the step surface and an angle formed between the beveled surface and the step side surface are both greater than 90°, a height of the beveled surface along an axial direction of the fixing ring is greater than a height of the step side surface along the axial direction of the fixing ring, so as to avoid the connecting portion.

10. The ventricular connection mechanism according to claim 1, wherein the clamping ring has an opening formed along a circumferential direction thereof, the inner diameter of the clamping ring is adjusted by adjusting a size of the opening, the sealing portion is provided with a notch corresponding to a position of the opening, the notch is provided on a side of the sealing portion away from the accommodating hole, so that when the inner diameter of the clamping ring decreases, the sealing portion at the notch is capable of being deformed toward the accommodating hole.

11. The ventricular connection mechanism according to claim 10, wherein an outer peripheral surface of the sealing portion is provided with a first flange extending radially outward from the sealing portion, the first flange is spaced from a groove bottom surface of the first annular groove to form a first slot, at least a portion of the clamping ring is provided in the first slot, the notch is provided on the first flange and extends along a circumferential direction of the suture ring, and the notch extends through both opposite sides of the first flange along an axial direction of the sealing ring.

12. The ventricular connection mechanism according to claim 1, wherein the clamping assembly further comprises two connecting arms connected to the clamping ring, the two connecting arms are capable of moving toward or away from each other, so as to adjust the inner diameter of the clamping ring, each connecting arm has a support pillar and an extension portion, the support pillar extends along an axial direction of the clamping ring, one end of the support pillar is connected to the clamping ring, the extension portion extends from an end of the support pillar away from the clamping ring in a direction away from the clamping ring along a radial direction of the clamping ring, the suturing portion is formed with a first avoidance groove in communication with the first annular groove, ends of support pillars of the two connecting arms that are connected to the clamping ring are accommodated in the first avoidance groove, and the extension portion is located outside the first avoidance groove.

13. The ventricular connection mechanism according to claim 12, further comprising a locking assembly, wherein the locking assembly comprises a fixing base, a moving base, and a locking member, the fixing base and the moving base are connected to two extension portions of the two connecting arms, respectively, one end of the locking member is connected to the fixing base, and another end of the locking member is capable of being connected to or separated from the moving base;
and/or, the first extension portion and the second extension portion are spaced apart from the suture ring by a distance in an axial direction of the suture ring.

14. The ventricular connection mechanism according to claim 1, wherein the suture ring is made of soft rubber, the ventricular connection mechanism further comprises a suturing cloth covering a surface of the suture ring and a surface of the clamping ring.

15. A ventricular assist system, comprising a ventricular assist device and the ventricular connection mechanism according to any one of claims 1 to 14, wherein the ventricular assist device comprises an inlet pipe extending through the accommodating hole.
